# EUROPEAN PATENT APPLICATION

(11) **EP 4 811 375 A1**
(43) Date of publication of application: **23.09.2026**
(21) Application number: 26165464.4
(22) Date of filing: 17.03.2026
(51) Int. Cl.: G16H 20/40

(54) **RADIOTHERAPY GRAPHICAL USER INTERFACES**

(30) Priority: 21.03.2025 US 202519087322
(71) Applicant: Varian Medical Systems, Inc., Palo Alto, CA 94304 (US)
(72) Inventor: SPATOLA, Brian, Palo Alto, 94304 (US); VOJAN, Filip, Palo Alto, 94304 (US)
(74) Representative: Mathisen & Macara LLP

(57) **Abstract**

Embodiments discussed herein provide methods (300) and systems (240) for enhancing usability and efficiency in radiotherapy and/or radiotherapy setup workflows through an intuitive graphical user interface (GUI) integrated into the gantry (224) of a radiotherapy machine (241). One method (300) includes presenting (310) a set of icons (406-432) on the display screen (226), where each icon (406-432) corresponds to instructions for adjusting at least one attribute of the radiotherapy machine (241) prior to treatment. Upon receiving (320) an input indicating that the instructions have been satisfied, the processor presents (330) a visual indicator on the display screen (226), confirming that the machine (241) is aligned for the treatment. The processor further configures (340) the adjusted attribute as a default attribute, ensuring consistency and streamlining subsequent treatment sessions. This method (300) enhances precision, reduces setup time, and provides real-time feedback, improving the overall efficiency and accuracy of radiotherapy and/or radiotherapy setup workflows.

## Description

### TECHNICAL FIELD

This application relates generally to graphical user interfaces and software for radiotherapy treatment setup and machines.

### BACKGROUND

Radiation therapy, which utilizes ionizing radiation, is a localized treatment targeting specific tissues, such as cancerous tumors. Ideally, this therapy focuses on the planning target volume (PTV) or target organ, sparing surrounding healthy tissue from excessive radiation doses to minimize damage. To ensure the prescribed dose is accurately delivered to the PTV, the patient must be precisely positioned relative to the linear accelerator, typically using a movable treatment couch on a turntable assembly. Implementing radiation therapy is a complex process involving specific guidelines, protocols, and instructions followed by various medical professionals, including clinicians, technicians, device manufacturers, and treating physicians. Due to the hazards associated with radiation, it is crucial that all instructions are meticulously followed.

Conventional radiation therapy systems are often complex to operate, requiring extensive training for efficient workflow execution. Tasks such as patient positioning, system calibration, and machine adjustments involve numerous interactions with both the patient and the radiotherapy machine. The data needed for these setups is often voluminous and complex, typically displayed on a screen within the treatment room. However, these conventional methods can be inefficient, ineffective, or even dangerous. Conventional radiotherapy systems do not efficiently display the data needed for patient and machine setup. In some conventional systems and methods, the necessary information for an operator (e.g., radiotherapy technician or any other medical professional) is not presented in a manner that is intuitive and user-friendly, interrupting the adjustment process and proving to be ineffective and time-consuming. Some other conventional systems and methods often provide all the information via static and complex graphical user interfaces (GUIs). Unless an operator is highly experienced with a particular system, this can slow down the setup process and degrade the patient's experience.

### SUMMARY

In accordance with a first aspect of the invention, there is provided a computer-readable medium as defined by claim 1.

In accordance with a second aspect of the invention, there is provided a computer system as defined by claim 7.

In accordance with a third aspect of the invention, there is provided a computer-readable medium as defined by claim 12.

Optional features are defined by the dependent claims.

In radiotherapy treatment workflows, the complexity of aligning both the patient and the radiotherapy machine can lead to confusion among operators when interpreting positional numbers (e.g., attribute with which the patient must be aligned and/or machine attributes). This is especially exacerbated when these alignment numbers are displayed on the radiotherapy GUI. In some embodiments, after completing the initial alignment, operators must often rely on precise numerical readouts to make further adjustments or confirm alignment accuracy. However, these numbers are frequently offsets from arbitrary starting positions or represent cumulative adjustments across multiple axes, which can be difficult to track and interpret. This complexity increases the likelihood of errors, particularly in high-pressure environments where operators are managing multiple tasks simultaneously. Small misinterpretations of these numbers can lead to misalignments, affecting treatment precision and potentially compromising patient safety.

For the aforementioned reasons, a clear and intuitive system of displaying alignment numbers (radiotherapy machine and/or patient) is needed to reduce confusion and to ensure that further adjustments are easy to understand and error-free. The methods and systems discussed herein provide a method of revising a radiotherapy GUI, such that various alignment attributes are reset (also referred to herein as "greenlighted"). Resetting the GUI establishes a consistent and precise reference point for all positional adjustments and measurements throughout the treatment process. By resetting all (or at least a portion of) readouts-such as couch position, gantry angle, and patient alignment-to zero after the initial setup, zeroing simplifies subsequent adjustments, allowing operators to intuitively make precise movements relative to a standardized baseline. Zeroing also allows for alignment across sessions, enabling reproducibility and reducing setup time for repeat treatments. In high-stakes environments requiring precision and efficiency, zeroing provides a straightforward and reliable method for maintaining accuracy and minimizing the cognitive load on operators.

In one embodiment, the techniques described herein relate to a computer-readable medium including a set of instructions, that when executed, cause at least one processor to: present, on a display screen located on a gantry of a radiotherapy machine configured to implement radiotherapy treatment of a patient, a set of icons corresponding to a set of instructions associated with adjusting at least one attribute of the radiotherapy machine before the radiotherapy treatment starts; receive an input that the set of instructions have been satisfied; present, on the display screen, a visual indicator demonstrating that the radiotherapy machine is aligned for the radiotherapy treatment of the patient.

The input may be received via an interaction with the display screen.

The input may be received via a pendant of the radiotherapy machine.

The pendant may include a button for the input.

The visual indicator may be an additional icon displayed on the display screen.

The visual indicator may correspond to a change of a visual attribute for at least one icon within the set of icons.

The input may be received after aligning a marking on a patient with an indicator emitted on the patient.

The set of instructions may further cause the at least one processor to: identify an additional adjustment to the at least one attribute; and present the additional adjustment in terms of a default configuration and not an original radiotherapy configuration.

In another embodiment, the techniques described herein relate to a computer system including: a radiotherapy machine; and at least one processor in communication with the radiotherapy machine, the at least one processor configured to: present, on a display screen located on a gantry of the radiotherapy machine configured to implement radiotherapy treatment of a patient, a set of icons corresponding to a set of instructions associated with adjusting at least one attribute of the radiotherapy machine before the radiotherapy treatment starts; receive an input that the set of instructions have been satisfied; present, on the display screen, a visual indicator demonstrating that the radiotherapy machine is aligned for the radiotherapy treatment of the patient.

The input may be received via an interaction with the display screen.

The input may be received via a pendant of the radiotherapy machine.

The pendant may include a button for the input.

The visual indicator may be an additional icon displayed on the display screen.

The visual indicator may correspond to a change of a visual attribute for at least one icon within the set of icons.

The input may be received after aligning a marking on a patient with an indicator emitted on the patient.

The at least one processor may be further configured to: identify an additional adjustment to the at least one attribute; and present the additional adjustment in terms of a default configuration and not an original radiotherapy configuration.

In another embodiment, the techniques described herein relate to a computer-readable medium including a set of instructions, that when executed, cause at least one processor to: present, on a display screen located on a gantry of a radiotherapy machine configured to implement radiotherapy treatment of a patient, a set of icons corresponding to a set of instructions associated with adjusting at least one attribute of the radiotherapy machine before the radiotherapy treatment starts; receive an instruction to set a machine parameter to a default attribute; and configure the at least one attribute of the radiotherapy machine as the default attribute.

The instruction may be received via an interaction with the display screen.

The instruction may be received via a pendant of the radiotherapy machine.

The pendant may include a button for the instruction.

The methods discussed herein may assist the user in performing a radiotherapy setup workflow, by means of a continued and/or guided process of human-machine interaction. The systems discussed herein may be configured to assist the user in performing a radiotherapy workflow and/or a radiotherapy setup workflow, by means of a continued and/or guided process of human-machine interaction.

### BRIEF DESCRIPTION OF THE DRAWINGS

Non-limiting embodiments of the present disclosure are described by way of example with reference to the accompanying figures, which are schematic and are not intended to be drawn to scale. Unless indicated as representing the background art, the figures represent aspects of the disclosure.
**FIG. 1** illustrates components of a workflow-oriented radiotherapy system, according to an embodiment.
**FIG. 2A** illustrates a radiotherapy machine in a default, upright orientation, according to an embodiment.
**FIG. 2B** illustrates a radiotherapy machine in a rotated orientation, according to an embodiment.
**FIG. 3** is a flow diagram of a method for displaying radiotherapy icons on a radiotherapy machine, according to an embodiment.
**FIGS. 4A-4D** illustrate a radiotherapy machine displaying a set of icons and instructions, according to an embodiment.
**FIG. 5** illustrates reference markings on a patient, according to an embodiment.
**FIGS. 6A-6B** illustrate reference markings on a patient, according to an embodiment.

### DETAILED DESCRIPTION

Reference will now be made to some embodiments illustrated in the drawings, and specific language will be used here to describe the same. It will nevertheless be understood that no limitation of the scope of the embodiments of the methods and systems described herein is thereby intended. Alterations and further modifications of the features illustrated here, and additional applications of the principles of the embodiments of the methods and systems described herein as illustrated here, which would occur to a person skilled in the relevant art and having possession of this disclosure, are to be considered within the scope of the embodiments, methods, and/or systems described herein.

### System Architecture

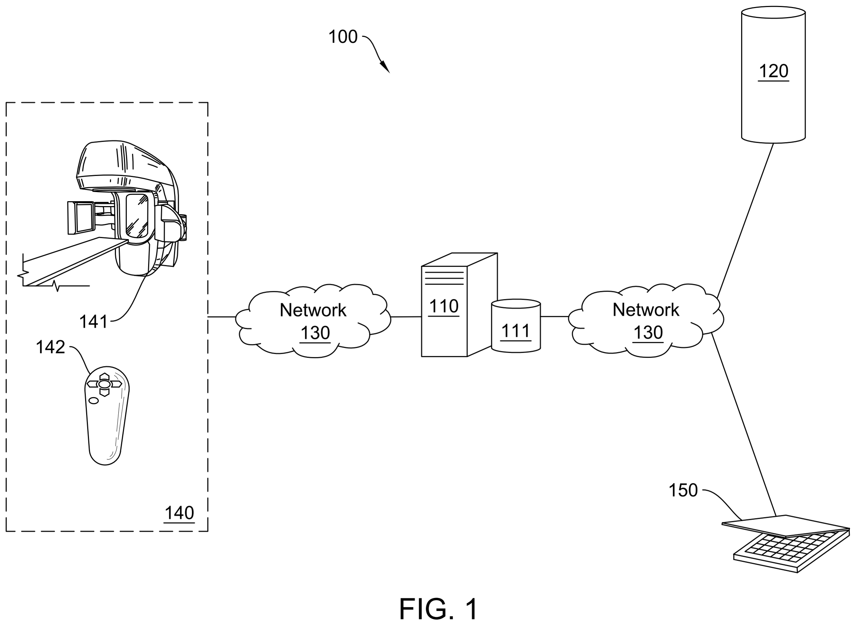

The methods described herein can be implemented using various computing devices/features described in **FIG. 1****.** Therefore, **FIG. 1** describes a non-limiting example of a computer environment where a server can perform the processes/methods described herein, such as retrieving, processing, and presenting radiotherapy treatment data, and presenting data for a graphical user interface (GUI) having workflow-oriented pages (or instances) on various displays screens.

**FIG. 1** illustrates various components of a system **100** for presenting various pages related to operation of a radiotherapy treatment, in accordance with an embodiment. The system **100** may include an analytics server **110,** a medical records database **120,** a radiotherapy system **140,** and a system administrator computer **150** or workstation. These features may communicate with each other over a network **130.** For example, the system **100** may present, optionally via the analytics server **110,** one or more pages/GUIs on the radiotherapy machine **141.** In another example, the system **100** may present, optionally via the analytics server **110,** a page presenting a live feed of a patient on one or more display devices, such as the display device on a gantry of the radiotherapy machine **141.** In general, any of the steps discussed herein as performed by the analytics server may be performed by the system in general.

The network **130** may include wired and/or wireless communications according to one or more standards via one or more transport mediums. Communication over the network **130** may be in accordance with various communication protocols, such as transmission control protocol and internet protocol (TCP/IP), user datagram protocol (UDP), and Institute of Electrical and Electronics Engineers (IEEE) communication protocols. The network **130** may further include wireless communications according to Bluetooth specification sets, or another standard or proprietary wireless communication protocol. The network **130** may further include communications over a cellular network, including, for example, a global system for mobile (GSM) communications, code division multiple access (CDMA), and enhanced data for global evolution network (EDGE). The examples of the network **130** may include, but are not limited to, private or public local area network (LAN), wireless LAN (WLAN), metropolitan area network (MAN), wide area network (WAN), and the Internet.

The analytics server **110** may be any computing device capable of performing the actions described herein. For instance, the analytics server **110** includes a processing unit and a non-transitory machine-readable storage medium. The processing unit includes a processor with a computer-readable medium, such as a random-access memory coupled to the processor. In some embodiments, the analytics server **110** executes algorithms or computer executable program instructions, which may be executed by a single processor or multiple processors in a distributed configuration. The instructions allow the processor to implement the functionality described herein. The analytics server **110** may be configured to interact with one or more software modules of a same or a different type operating within the system **100.**

Non-limiting examples of the processor may include a microprocessor, an application specific integrated circuit, and a field programmable object array, among others. The analytics server **110** may be capable of executing data processing tasks, data analysis tasks, and valuation tasks. Non-limiting examples of the analytics server **110** may include a desktop computer, a server computer, a laptop computer, a tablet computer, and the like. For simplicity, **FIG. 1** depicts a single-server computing device functioning as the analytics server **110.** However, some embodiments may include a plurality of server computing devices capable of performing various tasks described herein.

Implementation of the methods described herein is not limited to the system architecture depicted in **FIG. 1****.** In alternative embodiments, the analytics server **110** may be an embedded computing device disposed within the radiotherapy machine **141.** In some embodiments, the analytics server **110** may be a plurality of computing devices operated locally and/or remotely. In various embodiments, the analytics server **110** may be operated through a cloud service (e.g., network, internet). The cloud service may be performed in accordance with various communication protocols such as TCP/IP, UDP, and IEEE communication protocols. The analytics server **110** may utilize a database, such as a local database **111,** to store and/or retrieve various data described herein. For instance, the analytics server **110** may store different data corresponding to the different pages within the local database **111.** The page may be displayed on a display screen associated with the radiotherapy system **140.**

For instance, the analytics server **110** may display a page having a live feed of the patient before the treatment has begun and/or may display various pages describing how to position the patient on the couch and/or how to adjust the radiotherapy machine **141.** Even though some embodiments describe the analytics server **110** displaying various pages on a display screen attached to the radiotherapy machine **141** (e.g., located on the gantry of the radiotherapy machine **141),** it is expressly understood that the analytics server **110** may display different pages described herein on a display screen located anywhere within the treatment room, such as located on the wall of the treatment room or on any electronic device within the treatment room. In some configurations, the analytics server **110** may display the pages on the system administrator computer **150** or workstation.

The local database **111** may also store data corresponding to the radiotherapy machine **141** (e.g., default position of the radiotherapy machine **141,** and/or current position of the radiotherapy machine **141,** such as the orientation of the bed/couch and/or gantry).

If the analytics server **110** receives a request from the radiotherapy system **140** to provide the current position/orientation of the radiotherapy machine **141,** the analytics server **110** may query the local database **111** and may retrieve the corresponding data. Additionally, or alternatively, the analytics server **110** may also communicate with various sensors associated with the radiotherapy system **140** to retrieve machine data and parameters. The analytics server **110** may then use the methods/systems described herein to instruct the radiotherapy machine **141** to adjust the positioning of the radiotherapy machine **141** to the default position of the radiotherapy machine **141** or another position of the radiotherapy machine, or to display one or more pages instructing a technician to adjust the radiotherapy machine **141** and/or the patient.

The local database **111** may also store data associated with different users of the radiotherapy system **140.** In a non-limiting example, the user data may include the user's login (e.g., sign-in, credentials) information and authorization levels. For example, the analytics server **110** may store, using the local database **111,** the user's name and password. The analytics server **110** may allow the user to log in to the radiotherapy machine **141,** and/or the system administrator computer **150.** If the user logs-in into the system, the analytics server **110** may adjust what the user is able to view, adjust, and control based on the user's authorization level. The analytics server **110** may conceal the patient's medical information that is not relevant to the radiation treatment from the medical technician. In another example, the analytics server **110** may conceal all of the patient's medical information if a machine technician signs-in.

The analytics server **110** may also utilize one or more other databases, such as the medical records database **120,** to store and/or retrieve various data described herein. The databases herein can be configured as one or more databases storing the data, and the disclosure is not intended to be limited to a particular number or location of databases. The analytics server **110** may instruct the medical records database **120** to store patient data (e.g., patient name, patient machine alignment information) associated with a patient identifier (e.g., patient's name, patient's profile image). The analytics server **110** may then instruct the medical records database **120** to populate a dataset corresponding to a patient and display the profile image of the patient. If the analytics server **110** receives a request from the radiotherapy system **140** to display the data associated with the patient identifier, the analytics server **110** may query the medical records database **120** and may retrieve the corresponding dataset. The analytics server **110** may then use the methods/systems described herein to dynamically display the patient data in accordance with the patient identifier.

The medical records database **120** may also include a radiotherapy treatment file associated with the patient identifier. As used herein, the radiotherapy treatment file refers to all data associated with a patient's radiation therapy treatment and is not limited to a particular step or information. The radiotherapy treatment file may also be retrieved from the radiotherapy system **140,** the medical records database **120,** and/or the system administrator computer **150.** The radiotherapy treatment file may include the treatment data associated with a patient. The radiotherapy treatment file may be associated with a patient identifier and may also be updated after a patient has completed treatment. For example, after the patient has completed a treatment session, the medical records database **120** may retrieve the duration of the treatment session from the radiotherapy system **140** and update the radiotherapy file to include the duration of the treatment session. Even though aspects of the embodiments described herein discuss radiotherapy treatment as a file, the radiotherapy treatment file represents a collection of the patient's medical and treatment data, which may be stored in different files. For brevity, the present disclosure refers to the patient's data as a radiotherapy treatment file.

The radiotherapy treatment file may include data for treatment plans for one or more patients where each treatment plan is specific to a single patient. For instance, each treatment plan is uniquely created for each patient and corresponds to the patient's unique attributes (e.g., physical attributes of the patient and the patient's unique condition to be treated). In some configurations, each treatment plan for each patient may itself include multiple files.

The analytics server **110** may retrieve treatment data associated with a patient that is stored within the patient's radiotherapy treatment file(s). As described herein, the analytics server **110** may analyze the treatment data and may display various features and graphical components described herein. While the medical records database **120** may contain treatment data (radiotherapy treatment files) associated with multiple patients, the methods described herein are implemented such that various pages and graphical features described herein are specific to the particular patient being treated.

In some configurations, the analytics server **110** may retrieve radiotherapy treatment files associated with multiple patients. The analytics server **110** may then receive a selection by an operator (e.g., technician) of a patient to be treated. As a result, the analytics server **110** identifies the radiotherapy treatment file associated with the selected patient and customizes the graphical components described herein for the selected patient.

The analytics server **110** may also retrieve and instruct the medical records database **120** to store patient data associated with the patient from the medical records database **120,** the radiotherapy system **140,** and/or the system administrator computer **150.** For instance, the medical records database **120** may include patient data (e.g., previously populated by the analytics server **110** and/or periodically retrieved from a third-party data source). If a patient is selected, the analytics server **110** may query and retrieve patient data from the medical records database **120** and provide the retrieved patient data. For instance, the analytics server **110** may display the patient's profile image when the patient is selected, providing an opportunity to verify the correct patient was selected.

The analytics server **110** may receive treatment data associated with a patient from the medical records database **120,** the treatment data may further include at least a patient identifier. The analytics server may receive radiotherapy treatment file associated with one or more patients from the medical records database **120.** The radiotherapy treatment file may refer to a file having data associated with a process in which a medical team (e.g., radiation oncologists, radiation therapist, medical physicists, and/or medical dosimetrists) plan the appropriate external beam radiotherapy or internal brachytherapy treatment techniques for a patient.

The data within the radiotherapy file is not limited to the external radiation therapy as other treatments may impact the radiation therapy, such as medical oncology treatment (chemotherapy), interventional oncology treatment (e.g. cryotherapy, microwave therapy, or embolic therapy) or other non-treatment procedures, such as labs and appointments with other medical professionals. The radiotherapy treatment file may include data specific to one or more patient's radiotherapy treatment. The radiotherapy treatment file may include any combination of one or more of: a patient identifier, patient's electronic health data records, medical images (e.g., CT scans, 4D CT Scans, MRIs, and x-ray images), treatment-specific data (e.g., arc information or treatment type), target organ (e.g., specification and location data to identify the tumor to be eradicated), treatment plan, etc. Additional examples may include non-target organs, dosage-related calculations (e.g., radiation dose distribution within an anatomical region of the patient), and/or radiotherapy machine specific information (e.g., couch-gantry orientations, machine trajectory, control points, dose distributions, and/or arc information).

The analytics server **110** may use the patient identifier within the radiotherapy treatment file to identify a particular patient and retrieve additional information regarding said patient. For instance, the analytics server **110** may query the medical records database **120** to identify medical data associated with the patient. For instance, the analytics server may query data associated with the patient's anatomy, such as physical data (e.g., height, weight, and/or body mass index) and/or other health-related data (e.g., blood pressure or other data relevant to the patient receiving radiotherapy treatment). The analytics server **110** may also retrieve data associated with current and/or previous medical treatments received by the patient (e.g., prior treatment fractions, or data associated with the patient's previous surgeries).

The analytics server **110** may analyze the data received and generate additional queries accordingly. For instance, the analytics server **110** may retrieve data associated with one or more medical (or other) devices needed for the patient. The analytics server may retrieve data indicating that the patient suffers from a respiratory medical condition. As a result, the analytics server **110** may generate and transmit a query to the radiotherapy machine **141,** or the system administrator computer **150** to identify whether the patient uses/needs a ventilator.

If necessary, the analytics server **110** may also analyze the patient's medical data records to identify the needed patient attributes. For instance, the analytics server **110** may query a database to identify the patient's BMI. However, because many medical records are not digitalized, the analytics server **110** may not receive the patient's BMI value using simple query techniques. As a result, the analytics server **110** may retrieve the patient's electronic health data and may execute one or more analytical protocols (e.g., natural language processing) to identify the patient's body mass index. In another example, if the analytics server **110** does not receive tumor data (e.g., end-points) the analytics server **110** may execute various image recognition protocols and identify the tumor data.

Another example of a patient attribute may include specific tumor locations. More specifically, this data may indicate the primary tumor location with respect to the patient's centerline. This data may be inputted by the treating oncologist or may be analyzed using various image recognition or segmentation methods executed on the patient's medical images.

Another example of a patient attribute may include whether the patient uses prosthesis (e.g., hip or femoral head prosthesis). This attribute may result in a change of the patient's treatment (e.g., patients with these conditions might require a special treatment).

The analytics server **110** may use various application-programming interfaces (APIs) to communicate with different features described herein. As used herein, an API refers to a computing interface that uses connector programming code to act as a software intermediary between at least two computing components/features described herein. The API may automatically and/or periodically transfer various calls, instructions, and/or requests among different features of the system **100.** Using different APIs, the analytics server **110** may automatically transmit and/or receive calls and instructions.

Additionally, or alternatively, the analytics server **110** may use a content delivery network (CDN) to ensure data integrity when communicating with different features described in the system **100.** As described herein, a CDN refers to a distributed delivery network of proxy servers/nodes that uses multi-layered delivery methods/systems to transmit data (e.g., Akamai). The analytics server **110** may use a CDN when communicating various calls/instructions with the network **130** and/or the local database **111.**

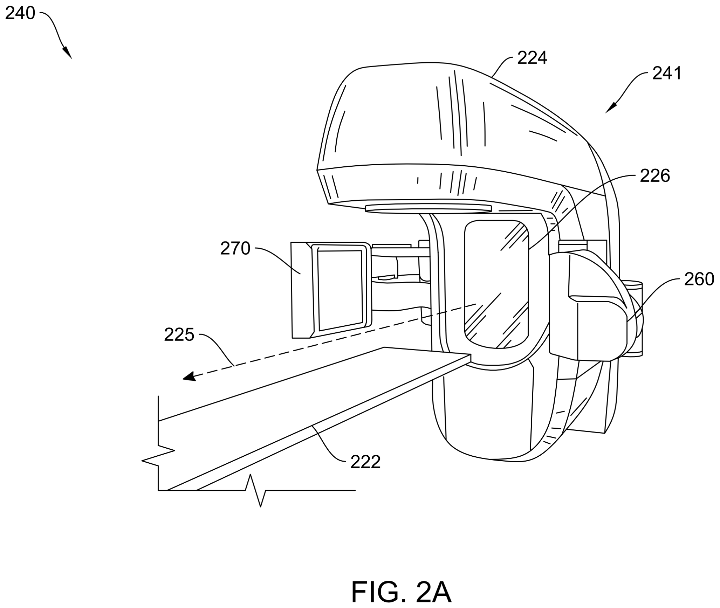

The radiotherapy machine **141** may also include a couch (shown as couch **222** in **FIG. 2A****).** to align the patient in a designated position before the treatment begins. The designated position may be identified, calculated, and/or retrieved by the analytics server **110.** For example, the analytics server **110** may retrieve patient data from the medical records database **120,** analyze the data, and utilize the analyzed data to position the patient on the couch. In some embodiments, the doctor identifies how to align the patient on the couch to optimize the treatment therapy. The radiotherapy machine **141** directs radiation at specified locations of the patient resting on the couch during treatment. The specified locations may be selected by the technician operating the radiotherapy machine **141,** the pendant **142,** and/or the system administrator computer **150.** The analytics server **110** may also specify the locations on the patient to direct the radiation.

The radiotherapy machine **141** may also include an x-ray emitter and an x-ray receiver. The x-ray emitter may be disposed on a gantry of the radiotherapy machine **141** and may be configured to provide x-ray (e.g., a penetrating form of high-energy electromagnetic radiation) waves. The x-ray emitter emits x-ray waves to the patient who is positioned on the couch. The x-ray receiver may be disposed opposed to the x-ray emitter. The x-ray waves received by the x-ray receiver generate an imprint (e.g., image) of the patient's internal anatomy. Although the example embodiment recites the use of x-ray imaging, an alternative configuration for the radiotherapy machine may include an additional or other medical imaging apparatus (e.g., fluoroscopy apparatus, MRI, etc.).

The radiotherapy machine **141** may also include a set of cameras (e.g., camera on an end of the couch, gantry camera, ceiling camera, or other cameras placed within the radiotherapy room). The analytics server **110** may retrieve a live feed of the couch (e.g., with or without the patient) from the set of cameras and provide it to a display screen disposed on the radiotherapy machine **141.** The set of cameras may also directly communicate with the radiotherapy system **140.**

The radiotherapy machine **141** may also include a gantry that may be in communication with the analytics server **110.** If the radiotherapy machine **141** is in operation, the gantry may rotate relative to the radiotherapy machine **141** to begin the treatment of the patient. The analytics server **110** may use the live feed of the set of cameras to control and/or stop the movement of the gantry. During operation, the analytics server **110** may instruct the display screen of the radiotherapy machine **141** to display the live feed of the set of cameras and/or a designated page to the display screen of the radiotherapy machine **141.** In various embodiments, the analytics server **110** may not instruct the display screen of the radiotherapy machine **141** to activate the display screen of the radiotherapy machine **141** during operation. In some embodiments, the display screen of the radiotherapy machine **141** may be a touch screen and may control the pages generated by the analytics server **110** through the display screen of the radiotherapy machine **141.**

The radiotherapy machine **141** may have a default home position. Before the patient receives treatment, the radiotherapy machine **141** may be reset to a position that is ergonomically desirable for the patient. The default home position may also be optimized to receive a new patient or allow the patient to more easily get off the couch.

As discussed in greater detail herein, the radiotherapy machine **141** may also be controlled by the pendant **142.** The pendant **142** may be connected to the radiotherapy machine **141** through wired or wireless communications according to, for example, Bluetooth specification sets or another standard or proprietary wireless communication protocol. In various embodiments, the pendant **142** may be connected to the radiotherapy machine **141** through a wired connection or be integrated into the radiotherapy machine **141.** The pendant **142** may also be in communication with the analytics server **110.** The pendant **142** may adjust the positioning of the radiotherapy machine **141** through a selection of buttons that axially actuates the radiotherapy machine **141** (e.g., couch) towards a desired direction. The technician may also use the pendant **142** to initialize the radiotherapy machine **141,** and/or to have the radiotherapy machine **141** return to its home position.

The pendant **142** may also allow the technician to input patient information, which may be then communicated to the radiotherapy machine **141,** the analytics server **110,** and/or the medical records database **120.** The pendant **142** may be used to control one or more of the steps of the setup and/or treatment of the patient.

The pendant **142** may also include a touch pad (e.g., tactile sensor). The touch pad may be used to control various pages generated by the analytics server **110.** For example, the user may use the touch pad of the pendant **142** to control a page, generated by the analytics server **110,** to proceed through the workflow steps of the radiotherapy setup and/or treatment. In some embodiments, the page may be controlled through a touch screen on the radiotherapy machine **141** or elsewhere in the treatment room.

As discussed in greater detail herein, the radiotherapy system **140** may further include accessories that assist in the radiotherapy setup and/or treatment of the patient. The analytics server **110** may communicate with the radiotherapy system **140** to select which accessories are required. The analytics server **110** may also communicate with the medical records database **120,** and/or the system administrator computer **150** to determine which accessories are required. For example, a patient may require a specific accessory for their radiotherapy setup and/or treatment. The analytics server **110** may retrieve that the patient requires a specific accessory by accessing the medical records database **120** and communicate that information to the radiotherapy system **140.** The accessories may be in communication with the radiotherapy system **140.** For instance, as will be described below, the analytics server may use RFID tags to scan and identify the location and/or presence of various accessories. In some embodiments, the accessories are wired or otherwise integrated into the radiotherapy machine **141.**

The local database **111** associated with the analytics server **110,** the medical records database **120,** and the radiotherapy machine **141** are capable of storing information in various formats and/or encrypted versions. The information may include data records associated with various patient information, user preferences, a set of prompts (e.g., question, query, and inquiry), attributes associated with various pages to be generated by the analytics server **110,** and the like. The medical records database **120,** may have a logical construct of data files, which are stored in non-transitory machine-readable storage media, such as a hard disk or memory, controlled by software modules of a database program (e.g., structured query language (SQL)), and a database management system that executes the code modules (e.g., SQL scripts) for various data queries and management functions.

The system administrator computer **150** may represent a computing device operated by a system administrator. The system administrator computer **150** may communicate with the analytics server **110.** The system administrator computer **150** may be configured to display various analytic metrics where the system administrator can monitor gantry movement, patient information, and modify various thresholds/rules described herein. The system administrator computer **150** may be configurable to display certain analytics metrics when specific thresholds/rules have been exceeded. For example, the system administrator computer **150** may be alerted if a patient has moved a specified amount during treatment. The analytics server **110** may allow the system administrator computer **150** to also control and/or override the settings of the radiotherapy machine **141,** and/or the pendant **142.** For instance, an administrator may revise the minimum distance threshold and/or customize any feature on the pages described herein (e.g., move features within the page, color, font, shape, size, or the order of display for one or more pages).

The analytics server **110** may configure the system administrator computer **150** to review any and/or all commands made through the radiotherapy system **140** at specified process steps. The system administrator computer **150** may then allow or reject the commands made through the radiotherapy system **140.** For example, before the technician starts the patient treatment using the radiotherapy machine **141,** the analytics server **110** may first prompt the system administrator computer **150** to review the radiotherapy machine **141** parameters for approval. Once approved, the technician may then control the radiotherapy machine **141.**

The analytics server **110** may configure the system administrator computer **150** to review any and/or all reading and/or (over)writing of patient data to and/or from the medical records database **120.** For example, before the technician may (over)write patient information collected by the radiotherapy machine **141,** the analytics server **110** may first prompt the system administrator computer **150** to review the patient information before it is stored in the medical records database **120.**

**FIG. 2A** illustrates, as described above, a radiotherapy system **240** including a radiotherapy machine **241** that includes a gantry **224** that rotates about a center axis **225** of the radiotherapy machine **241.** In some embodiments, the radiotherapy system **240** may be substantially similar to the radiotherapy system **140** of **FIG. 1****.** Likewise, the radiotherapy machine **241** may be substantially similar to the radiotherapy machine **141** of **FIG. 1****.** The radiotherapy machine **241** may further include a display screen **226** positioned on or within the gantry **224.** The display screen **226** may be configured to display for view to a user (e.g., a technician, patient, and/or a medical practitioner) relevant information related to the radiotherapy procedure before, during, and/or after the radiotherapy procedure.

The radiotherapy machine **241** may also include an imager **270** and an emitter **260** for administering the radiotherapy to a patient. The radiotherapy system **240** may additionally include a couch **222,** upon which the patient may be placed during the administration of the radiotherapy. In some embodiments, the couch **222** may be translated laterally relative to the radiotherapy machine **241** (e.g., along the center axis **225)** and/or rotated axially relative to the center axis **225** of the radiotherapy machine **241.** Likewise, in some embodiments, the radiotherapy machine **241** may be rotated axially about the center axis **225,** and relative to the couch **222,** before, during, and/or after the administration of the radiotherapy treatment.

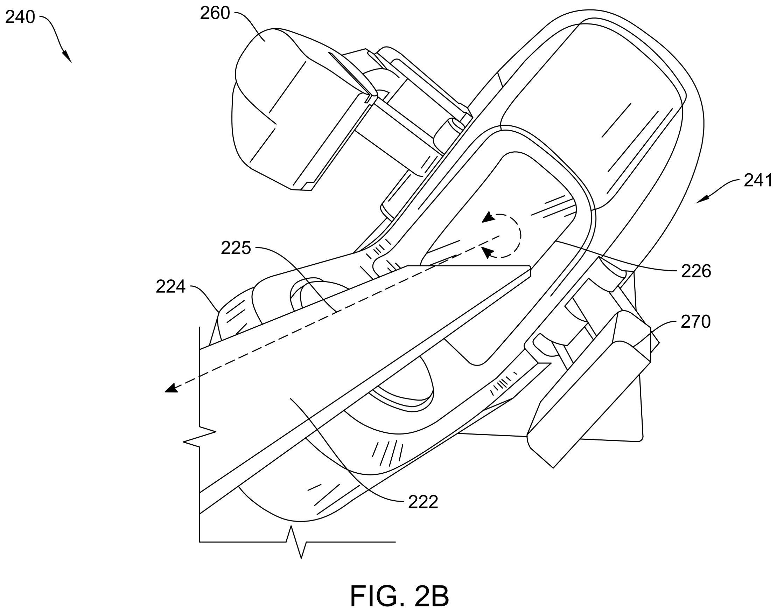

For example, as illustrated in **FIG. 2B****,** the radiotherapy machine **241** is shown in a rotated position about the center axis **225.** Relative to the default position of the radiotherapy machine **241** (as shown in **FIG. 2A****),** the radiotherapy machine **241** in **FIG. 2B** is shown at a position that is rotated about 135.5° counter-clockwise. Because the display screen **226** is coupled to the gantry **224** of the radiotherapy machine **241,** the display screen **226** is also rotated from a default, upright position (as shown in **FIG. 2A****)** at about 135.5° counterclockwise.

Using the methods and systems discussed herein, one or more processors (e.g., the analytics server discussed in **FIG. 1****)** may provide a visual indication when the absolute values of a selected group of readouts reach a desired or preset value, enabling the operator to quickly determine if key machine components, such as the gantry, collimator, and couch rotations, are set to zero or another predefined starting position. This can streamline the patient setup process by eliminating the need for operators to manually scan multiple readouts, reducing cognitive load and potential errors. For example, when the system detects that certain positional values align with the preset condition, it may highlight an indicator, change the color of relevant readouts, or present a confirmation message to visually notify the operator. This concept is sometimes referred to as "green lighting" and simplifies workflow efficiency, ensuring that the machine is correctly positioned before initiating further treatment steps.

Moreover, using the methods and systems discussed herein, one or more processors may "zero out" the radiotherapy machine during the treatment setup process. The concept of zeroing in radiotherapy, as used herein, may refer to the process of establishing a reference point, or "zero point," within the treatment system that serves as the baseline for all subsequent positional adjustments and measurements. This zero point can be applied to various axes or components, such as the treatment couch, gantry, or beam positioning system, ensuring that all movements and alignments are calculated relative to this consistent origin. The methods and systems discussed herein also dynamically revise the GUI discussed herein to allow the operator to easily understand how to make adjustments to the patient and/or the radiotherapy machine.

By defining a standardized reference, zeroing simplifies the workflow, reduces potential errors during setup, and enhances the precision and efficiency of radiotherapy procedures. For example, once a patient is aligned with the radiotherapy machine's isocenter or another predefined treatment position, the system can reset all positional readouts to zero, enabling operators to make precise adjustments without the need to interpret offsets from arbitrary starting points.

Zeroing may involve a mutual interaction between the operator and the system to confirm the alignment and establish the reference point. The process can be dynamically implemented through an interface or a dedicated control, such as a button on the pendant or a console computer/monitor, that recalibrates the positional data once activated. This recalibration ensures consistency across the treatment session by providing an intuitive framework for subsequent adjustments. For instance, if additional shifts are required after zeroing, the movements can be measured and displayed relative to the baseline, maintaining accuracy throughout the workflow.

This approach not only simplifies adjustments, such as couch movements or gantry rotations, but also ensures that the treatment setup aligns seamlessly with the planned parameters, reducing operator workload and improving treatment outcomes.

The systems and methods discussed herein discuss multiple embodiments and concepts including zeroing and greenlighting.

Zeroing, as used herein, may refer to the process of establishing a reference point, or "zero point," within the treatment system that serves as the baseline for all subsequent positional adjustments and measurements (e.g., machine attributes). This zero point can be applied to various axes or components, such as the treatment couch, gantry, or beam positioning system, ensuring that all movements and alignments are calculated relative to this consistent origin. By resetting all positional readouts to zero after the initial setup, zeroing simplifies subsequent adjustments, allowing operators to make precise movements relative to a standardized baseline. This approach enhances precision and efficiency in radiotherapy procedures, reducing potential errors during setup and ensuring that the treatment setup aligns seamlessly with the planned parameters.

The concept of zeroing can be performed at any time based on receiving an instruction from the operator. In one non-limiting example, during the initial setup of a radiotherapy session, the operator positions the patient on the treatment couch and aligns the patient with the radiotherapy machine's isocenter. Once the initial alignment is complete, the operator can issue an instruction to zero the machine. This action resets all positional readouts, such as couch position, gantry angle, and/or patient alignment, to zero. This zeroing establishes a consistent reference point for all subsequent adjustments, ensuring that any further movements are made relative to this standardized baseline. This simplifies the process of making precise adjustments and reduces the likelihood of errors during the treatment session.

In another non-limiting example, during a radiotherapy session, the operator may need to make adjustments to the patient's position or the machine's settings due to unexpected movements or changes in the treatment plan. For instance, if the patient shifts slightly on the couch, the operator can realign the patient and then issue an instruction to zero the machine again. This action resets the positional readouts to zero based on the new alignment, allowing the operator to make further adjustments with reference to the updated baseline. This ensures that the treatment remains accurate and consistent, even when adjustments are needed mid-session.

Greenlighting, as used herein, may refer to a concept used to provide a visual indication when the absolute values of a selected group of readouts reach a desired or preset value. This visual indicator, often in the form of a green light or a similar visual cue, enables the operator to quickly determine if key machine components, such as the gantry, collimator, and/or couch rotations, are set to zero or another predefined starting position. Greenlighting streamlines the patient setup process by eliminating the need for operators to manually scan multiple readouts, reducing cognitive load and potential errors. When the system detects that certain positional values align with the preset condition, it may highlight an indicator, change the color of relevant readouts, or present a confirmation message to visually notify the operator. The visual indicator may automatically indicate that all (or sometimes a certain defined portion of) the setup (whether machine or patient) has been accomplished successfully and the patient and the machine are ready for the treatment to start.

The zeroing and greenlighting concepts are designed to improve the accuracy and efficiency of radiotherapy treatments by providing clear and intuitive systems for displaying alignment numbers and ensuring that further adjustments are easy to understand and error-free.

In some embodiments, zeroing and greenlighting can be performed together to enhance the efficiency and accuracy of radiotherapy treatments. For instance, when the machine reaches the desired alignment and displays the greenlight indicator, signaling that all key components such as the gantry, collimator, and/or couch rotations are correctly positioned, it can simultaneously reset the positional readouts to zero. This dual action ensures that the machine is not only ready for treatment but also establishes a consistent reference point for any further adjustments. By combining zeroing with greenlighting, operators can streamline the setup process, reduce cognitive load, and minimize the risk of errors, ultimately improving the precision and reliability of radiotherapy procedures. This integrated approach provides a straightforward and reliable method for maintaining accuracy and minimizing the cognitive load on operators.

Alternatively, the greenlighting and/or zeroing may be performed independently. For instance, during a radiotherapy session, the operator may first use the greenlighting feature to ensure that the machine components, such as the gantry, collimator, and/or couch rotations, are correctly positioned according to the treatment plan. The greenlight indicator provides a visual confirmation that the machine is ready for treatment. Later, if the operator needs to make additional adjustments to the patient's position or the machine's settings, they can independently perform the zeroing process. Moreover, even at a later time, the operator can instruct an additional zeroing. This independent use of greenlighting and zeroing allows for flexibility in the treatment setup process, ensuring that each step is performed accurately and efficiently without being dependent on the other. Accordingly, the zeroing can be done at anytime based on receiving an instruction from the operator. The greenlighting and zeroing may be performed independently at different times.

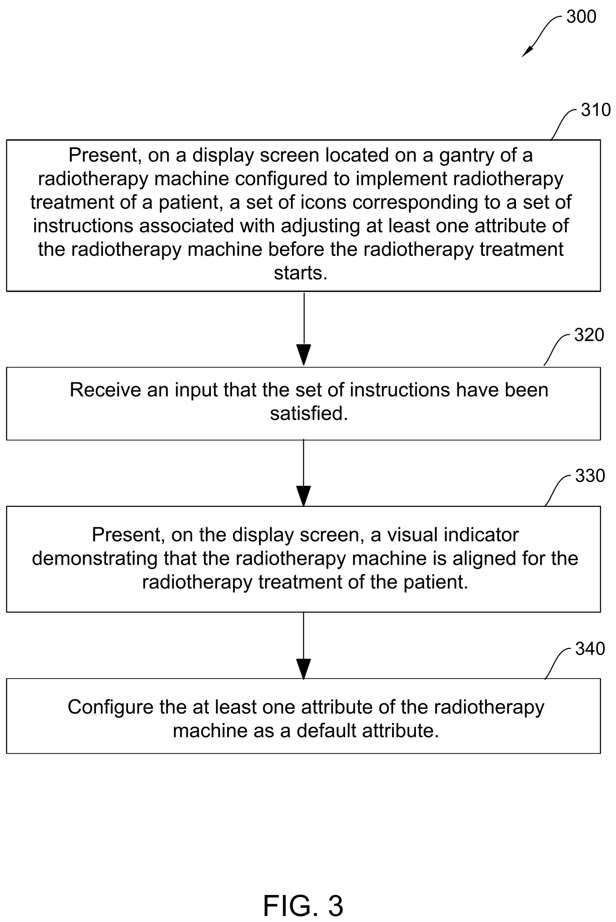

**FIG. 3** is a flowchart of an example method **300** for displaying one or more icons and instructions on a display screen coupled to a radiotherapy machine. The method **300** may be executed by one or more processors described herein, such as the analytics server **110,** the pendant **142,** the system administrator computer **150,** the radiotherapy system **140,** the radiotherapy machine **141,** etc. The one or more processors may execute instructions stored on a computer-readable, non-transitory medium, which may cause the one or more processors to execute steps of the method **300.** While the steps of the method **300** are shown in a specific order, it should be understood that the method **300** may comprise more, fewer, and/or different steps than those depicted in **FIG. 3****.** Likewise, the order of the steps of the method **300** is shown in **FIG. 3** for illustrative purposes. However, it is understood that the steps of method **300** may be performed in any number of configurations or orders without departing from the scope of the systems and methods described herein.

At step **310,** one or more processors may present, on a display screen located on a gantry of a radiotherapy machine configured to implement radiotherapy treatment of a patient, a set of icons each corresponding to a set of instructions associated with the radiotherapy treatment and/or radiotherapy setup.

As discussed herein, one or more processors may generate a set of tasks for configuring a radiotherapy treatment for a patient. Each task within the set may be associated with a specific stage of the radiotherapy treatment and/or radiotherapy setup, facilitating a structured and workflow-oriented approach to treatment setup, planning and/or execution. In some embodiments, the one or more processors may first retrieve a radiotherapy file (RT file) associated with the patient in order to identify the patient's treatment data.

As used herein, the RT file may refer to a comprehensive collection of data associated with a patient's radiotherapy treatment, encompassing all information necessary for setup, planning, executing, and/or monitoring the treatment process. In some embodiments, the RT file may include patient identifiers, such as names and demographic details, along with imaging data like CT scans, 4D CT scans, MRIs, and x-rays that provide anatomical details for treatment planning. The RT file may also contain detailed treatment plans, including beam trajectories, dose levels, arc information, and the type of radiotherapy being administered. Additionally, the RT file may specify target and non-target organ data to ensure accurate radiation targeting while minimizing exposure to healthy tissues.

Additionally, the RT file may include patient-specific attributes such as physical characteristics (e.g., height, weight, BMI) and medical history, as well as details about required accessories like immobilization devices or bolus materials. Session-specific data, such as couch positioning and adjustments from prior treatments, are also recorded to enable consistency across treatment sessions. The RT file may serve as a resource for the one or more processors, enabling the dynamic generation of workflow tasks (e.g., the values displayed for each icon), real-time updates to the GUI, and personalized treatment workflows. This structured approach allows the GUI to be populated with precise and efficient radiotherapy tailored to the unique needs of each patient. By leveraging the patient's RT file, medical records, and treatment data, the one or more processors may identify and define tasks tailored to the patient's unique attributes and treatment requirements.

The set of tasks may correspond to various stages of the radiotherapy treatment and/or radiotherapy setup, such as patient alignment, machine calibration, accessory verification, dose administration, post-treatment analysis, and the like. For instance, during the alignment stage, a task may involve verifying the patient's position on the treatment couch relative to the isocenter of the radiotherapy machine. The one or more processors may generate tasks to guide the operator in adjusting the couch and gantry to achieve the required alignment.

After the tasks and icons have been identified, the one or more processors may display the icons. In some embodiments, the display screen, integrated into the gantry, may provide a visually accessible/interactive interface that assists operators in efficiently navigating through treatment tasks without diverting attention from the patient or the machine. The GUI may display various icons where each icon corresponds to a specific task within the treatment and/or treatment setup workflow, such as patient alignment, machine calibration, and/or dose administration, and provides intuitive visual indicators to guide the operator through the sequential stages of the treatment and/or treatment setup process.

The set of icons may be radially arranged on the display screen to enhance accessibility and usability during operation. In this way, the icons (or at least a portion of the icons) can be displayed simultaneously. The radial configuration allows operators to easily identify and interact with individual icons regardless of the screen's orientation or the gantry's position. The radial arrangement is designed to optimize spatial organization and ensure that icons remain legible and logically grouped even as the gantry rotates. In some embodiments, the one or more processors may dynamically update the icons based on task completion, visually distinguishing completed tasks and highlighting the next actionable steps in the workflow. This arrangement, combined with real-time updates, improves operational efficiency by reducing cognitive load and enabling seamless progression through the radiotherapy treatment and/or radiotherapy setup process.

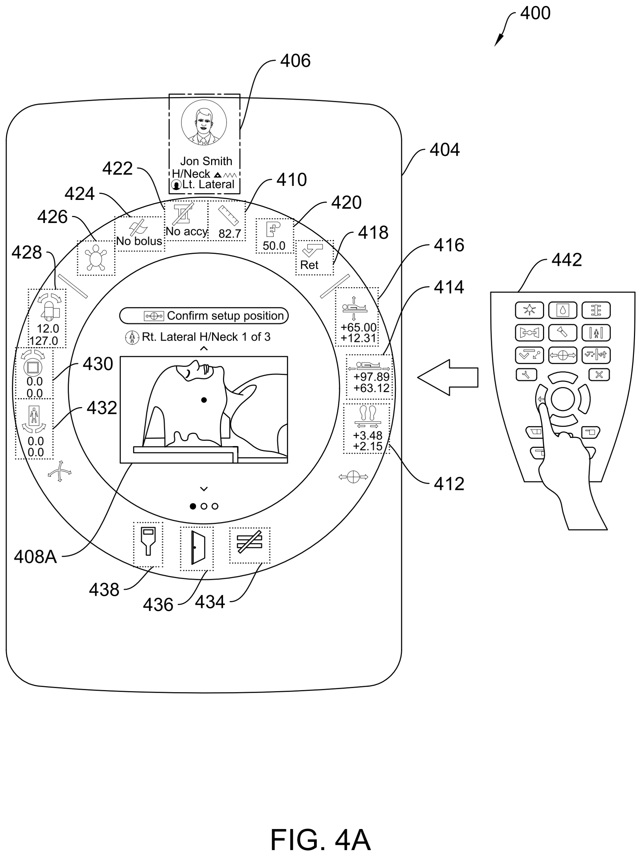

The radial arrangement may place the icons around a center point of the display screen (e.g., center portion **408** discussed in **FIGS. 4A-B****),** allowing the icons to be uniformly distributed across the display screen.

In some embodiments, the icons may be displayed radially with each icon positioned at an equal, substantially equal or close to equal, radius from the central point on the display screen. This uniform radial distance may provide a balanced icon layout, where each icon is equidistant or near equidistant from the center point of the display screen, making them easily accessible and visually consistent. Such a configuration minimizes cognitive effort for the operator, as icons are uniformly spaced, and their positions can be intuitively located within the circular arrangement.

The icons may form a full circle or a semi-circle around the central point, depending on the screen size, radiotherapy machine type, the number of icons, and the workflow requirements. In some embodiments, a full circle arrangement may be used for maximizing icon capacity while maintaining symmetry (e.g., when multiple tasks need to be displayed simultaneously). In contrast, a semi-circle arrangement can be used to emphasize a subset of tasks or to make space for additional interface elements, such as dynamic instruction displays or task-specific information. These configurations can adapt dynamically to the operator's needs, presenting icons in a visually organized manner while keeping all relevant information within the same page.

In embodiments where a full-circle arrangement is used, the central point of the display screen could be reserved for detailed task instructions, with the icons evenly distributed around it. In a semi-circle arrangement, the bottom half of the screen could be left open for visual indicators, such as task progress bars or alerts, while the upper semi-circle (and sometimes side portions) may contain the workflow icons. The radial design, whether a full or partial circle, ensures that the icons remain logically grouped and easy to access, even as the gantry or display rotates, maintaining operator efficiency and usability.

The layout of the radial display may allow the operator to easily locate and interact with each icon regardless of the gantry's rotation or the display's orientation. By organizing the icons radially, the system minimizes visual clutter while maintaining a logical grouping of the icons.

The icons displayed on the display screen may be grouped based on predefined attributes such as function, workflow stage, or type of information, and these groupings can be customized or modified to suit the preferences of different operators or clinics. For instance, icons related to patient information, such as demographic data or treatment specifics, may be grouped separately from icons representing machine setup tasks, such as couch alignment or gantry positioning. The predefined grouping criteria may allow for consistency across workflows while allowing for flexibility to adapt to varying clinical protocols or individual operator needs. Customization options enable clinics to tailor the grouping and presentation of icons based on their unique workflows, equipment configurations, or operational preferences. This adaptability can be used to ensure that the system remains intuitive and efficient, regardless of the user's specific requirements or the treatment environment.

In some embodiments, the icons may be arranged in a sequential order that reflects the typical (e.g., pre-defined) workflow of the treatment process, ensuring tasks are performed in the correct sequence and guiding the operator intuitively through each stage. As a result, the location of the icons may correspond to the order of the tasks. For instance, the icons may be arranged such that a subsequently displayed icon (when considering the display of the icons clockwise or counterclockwise depending on the setup of the icons) may correspond to the next task to be accomplished.

In a non-limiting example, the icon arrangement starts with a patient identification icon, placed at the beginning (most left or most right of the displayed icons) to allow the operator to verify the patient's identity and treatment plan, ensuring the correct individual is being treated. This foundational step minimizes the risk of errors and sets the stage for subsequent tasks. Following this, all other icons may be arranged in a clockwise manner, such that the operator can intuitively understand which task must be performed next.

This logical and workflow-oriented arrangement of icons ensures that operators are intuitively guided through the radiotherapy and/or setup process, reducing cognitive load and minimizing errors. The sequence of icons can also be dynamically adjusted by the analytics server based on real-time task completion or user inputs, providing flexibility to accommodate specific scenarios or operator preferences. This approach ensures that the GUI remains efficient, user-friendly, and aligned with the needs of the radiotherapy and/or radiotherapy setup workflow.

In addition to corresponding to a specific task, each icon may also be dynamically interactive, adapting to changes in the treatment environment and user inputs. This dynamic adaptation may be driven by the analytics server (or any other server/processors discussed herein), which monitors the progress of each task and transitions the GUI to display/highlight the next set of icons upon task completion. For instance, once the alignment of the patient is verified, the corresponding icon may be updated (e.g., visually by changing colors) to indicate completion, while the next icon highlights the verification of dose calibration. This sequential and responsive design ensures that the operator is guided seamlessly through the treatment and/or treatment setup workflow without needing to be directed to a new page and while viewing the status of other icons/tasks, enhancing efficiency and reducing setup time. Moreover, the distinct visual and functional characteristics of each icon-such as color changes, animations, or text overlays-serve as intuitive indicators of task status, enabling quick recognition and action by the operator.

Referring now to **FIG. 4A****,** a display of the icons on a display screen of a radiotherapy machine is presented, in accordance with one embodiment. The set of icons, in this embodiment **400A** are presented on a display screen **404** (similar to the display screen **226)** located on a gantry of a radiotherapy machine. As depicted, the icons **406-432** are displayed radially on the display screen **404,** though in other embodiments, the arrangement may be different. The display screen may also include a center portion **408A-C** that can display additional data needed or otherwise associated with different icons.

As depicted, the set of displayed icons includes an icon **406** that may include the patient's personally identifiable information, such as the patient's image, name, diagnoses, and other information that can identify the patient.

Different groupings of icons (or sometimes individual icons) may be arranged in accordance with a predefined order that may or may not correspond to the workflow of the patient's radiotherapy treatment and/or radiotherapy setup. For instance, in one embodiment, different tasks to be performed may be associated with a defined order. As a result, their corresponding icons may be arranged in that order.

In other embodiments, such as the depicted embodiment, the icons may be arranged in accordance with a grouping of the tasks. For instance, icons may be arranged based on whether they are associated with a patient setup category, radiotherapy accessory category, radiotherapy machine attribute category, rotational parameter category, interlocks, or a system status category. As depicted, the set of icons may be divided into three groups where the groups indicate a cluster of tasks to be performed. The cluster of tasks may have a common attribute and may be clustered based on the common attribute. For instance, one grouping or cluster of icons may indicate adjustments to be made to the patient (e.g., patient alignment) and another grouping of the icons may be directed to machine adjustment.

A first grouping of the icons may include patient alignment indicators (e.g., icons **412, 414,** and **416).** The icon **412** may indicate whether the patient needs to be laterally moved (e.g., moved to the left or right). Additionally, the set of icons may include an icon **414** that indicates whether the patient should be moved on the couch (e.g., toward the radiotherapy machine or away from the machine). Additionally, the set of icons may include an icon **416** that indicates whether the patient on the couch should be moved vertically (e.g., whether the couch should be moved up or down).

A second grouping of icons may include machine/treatment accessories and may include icons **410, 418, 420, 422,** and **424.** The icon **410** may correspond to a distance indicator measuring/indicating the distance between the radiation source and the patient's skin (source-to-skin distance, SSD). Additionally or alternatively, the set of icons may include an icon **418** that indicates whether an imager of the machine is extended. Additionally or alternatively, the set of icons may include an icon **420** that indicates the status of the imagers of the machine (e.g., how far the imagers are from the machine). Additionally or alternatively, the set of icons may include an icon **422** that indicates whether an accessory (and if so, which accessory) is needed to treat the patient. Additionally or alternatively, the set of icons may include an icon **424** that indicates whether a bolus is needed to treat the patient. Additionally or alternatively, the set of icons may include an icon **426** that indicates the patient orientation (e.g., whether the patient should be on their back with their head toward the machine or otherwise). Additionally or alternatively, the set of icons may include an icon **428** that indicates whether any adjustment to the gantry is needed. The icon **428** may include arrows indicating which direction the gantry should be turned or otherwise moved towards. In some embodiments, the icon **428** may also include an angle value indicating a desired position of the gantry.

Another grouping of the icons may include machine attribute icons that correspond to tasks related to adjusting the radiotherapy machine. This group may include icons **428, 430,** and **432.** The icon **430** may indicate whether any adjustments are needed to be made to the collimator. In some embodiments, the icon **430** may include a map of the collimator opening. Additionally, the set of icons may include an icon **432.**

In some embodiments, system administrators or operators may have the capability to adjust any attribute of the icons displayed on the display screen, including visual attributes such as color, font style, size, line thickness, shading, and the like. This flexibility allows the icons to be modified to align with institutional branding, operator preferences, or accessibility requirements. For instance, line thickness or font size can be increased to enhance visibility for users with visual impairments. These adjustments ensure that the interface remains both functional and user-friendly, tailored to meet the specific needs of the clinical setting.

The position of the icons on the display screen can also be adjusted by system administrators or operators to optimize usability and align with operator workflows. Icons can be repositioned within the radial arrangement or moved to alternative layouts, ensuring that the most frequently accessed icons are conveniently located. This customization enables the interface to adapt to specific clinic requirements or individual user preferences, improving efficiency during radiotherapy procedures and/or radiotherapy setup procedures. For instance, a clinic focusing on particular types of treatments and/or setups may prioritize the placement of certain icons for quick access, minimizing navigation time and enhancing operational precision.

In some embodiments, a system administrator or an operator can be authorized to add new ones to better reflect the workflow or accommodate updates to the radiotherapy process. This capability ensures that the interface remains relevant and streamlined, reducing visual clutter and focusing only on the tasks and information pertinent to the specific clinical scenario. For example, an icon can be added to the set of icons that indicates any combination of one or more of: whether a door of the treatment room is open, whether a predicted collision is going to occur, whether the pendant is properly connected to the radiotherapy machine, and the like.

The set of icons may also include an icon **438** indicating whether a pendant is properly connected to the radiotherapy machine, icon **438** indicating whether the treatment room door has been properly closed, and/or icon **434** indicating whether the machine parameters are or are not in the planned positions.

The icons displayed may be interactive, such that the operator can move along the icons and select (e.g., click, press, touch, or otherwise interact with different icons), e.g., using a pendant of the radiotherapy machine, such as the pendant **442.** For instance, the operator may use one or more buttons on the pendant **442** to navigate through the displayed icons and use another button to make a selection.

In some embodiments, the one or more processors may present a visual indicator on the display screen indicating that the subset of the set of instructions satisfies a threshold, and the subset of the set of instructions is displayed in a plurality of pages. The visual indicator may be designed to inform the operator that a subset of the set of instructions satisfies a predefined threshold. As used herein, the threshold may correspond to any attribute that is defined by a system administrator or an operator. For instance, the threshold may correspond to a number of instructions for the selected icon. In another example, the threshold may indicate a predefined progression of instructions that are designed to be separately and/or consecutively displayed. For example, when the subset of instructions exceeds the display capacity of a single page, the one or more processors organize and display the instructions across a plurality of pages, ensuring that all relevant information is accessible without overwhelming the interface or compromising usability.

In some embodiments where the subset of instructions contains numerous detailed steps, such as alignment adjustments, gantry configuration, or dose calibration, the one or more processors may distribute these steps across multiple pages. Moreover, the one or more processors may display a visual indicator informing the operator that the instructions are displayed on multiple pages. Non-limiting examples of a visual indicator may include visual navigation cues, such as pagination icons, scrolling indicators, toggle icons, or a progress bar, and the like. The visual indicator may allow the operator to seamlessly navigate between pages while ensuring clarity and task focus. For instance, the indicator may convey the number of pages and the position of the current page (among the set of pages) being displayed. This functionality ensures that the interface remains efficient and user-friendly, even when managing complex or large sets of instructions, ultimately improving workflow efficiency and treatment accuracy.

For instance, and still referring to **FIG. 4A****,** the one or more processors may determine that the selected icon includes more information than can be displayed on one page. As a result, the one or more processors may display the visual indicator **440A** indicating that the information displayed in the center portion **408A** includes multiple pages. The visual indicator **440A** indicates that there are three total pages (e.g., three dots) and further indicates that the currently displayed page is the first of three pages.

Referring back to **FIG. 3****,** at step **320,** the one or more processors may receive an input that the set of instructions have been satisfied. In some embodiments, the instructions may correspond to aligning the patient via various predefined alignment attributes. In some embodiments, the instructions may correspond to the operator aligning or adjusting the radiotherapy machine to defined machine settings/attributes (e.g., adjusting the couch elevation). The input may indicate and confirm that the operator has made the required adjustments/alignments.

In some embodiments, the input may be provided by the operator through various interaction mechanisms, such as selecting a confirmation button on the GUI and/or pressing a physical button on a pendant or a console. In some other embodiments, the one or more processors may retrieve data from an external source, such as the radiotherapy machine itself and/or other sensors within the treatment room. For instance, if the task provided to the operator is to move the couch to a defined elevation, the one or more processors may communicate with a processor of the radiotherapy machine and determine when the couch is moved. In another example, the one or more processors may communicate with a camera operated within the treatment room and determine if the patient has been moved and is not in a particular position.

The input received from the operator may indicate that the values of a selected group of machine readouts have reached a desired or preset value. Therefore, the input indicates that the instructions regarding adjusting the machine attributes (e.g., movement of the couch or gantry) has been followed and the machine attributes indeed match the desired attributes. Effectively, the operator may indicate that the machine has been aligned using the instructions provided. In some embodiments, upon receiving this input, the one or more processors may verify that the machine components, such as the gantry, collimator, and couch rotations, align with the predefined conditions.

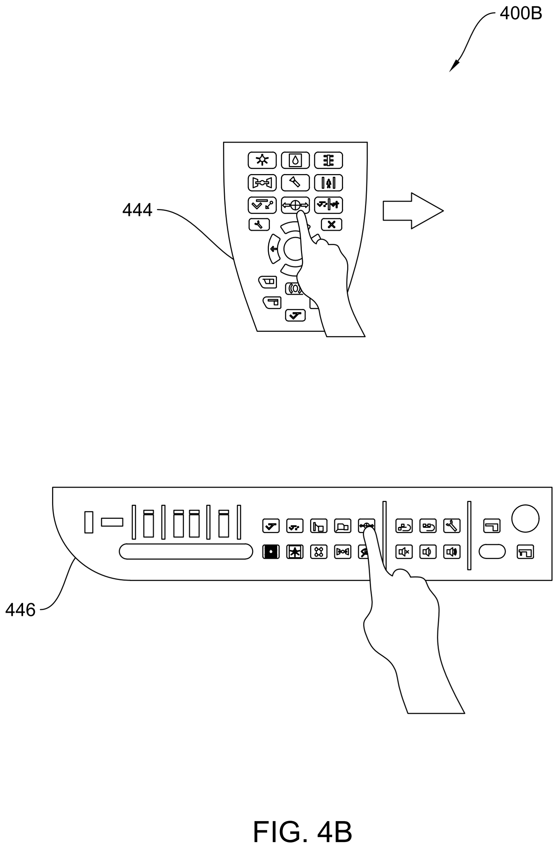

Referring now to **FIG. 4B****,** the embodiment **400B** depicts two different examples of the operator providing the input to the one or more processors indicating that a task has been completed or otherwise the instructions have been satisfied or followed. In the first example, the operator may use a dedicated button using the pendant (example **444).** In the second example, the operator (or another operator outside the treatment room) may use a console monitor **446** to indicate that the task has been accomplished, e.g., using a dedicated button. In some embodiments, the dedicated button may change its color when the confirming option is available (e.g., when the operator has the option to confirm alignment). This allows the operator to identify the dedicated button. The dedicated button may then change color after the operator has confirmed alignment of the patient and/or machine.

In some embodiments, the confirmation of whether the patient is properly aligned may be performed by aligning the patient with pre-generated marks or reference points. Reference marking in radiotherapy and/or radiotherapy setup may involve creating physical or visual markers (sometimes referred to as reference points) on the patient or their immobilization devices (accessories) to establish precise alignment points, ensuring consistent positioning across multiple treatment sessions. These markers may act as alignment guides, ensuring that the patient's position on the treatment couch matches the planned position from the imaging and treatment planning stages.

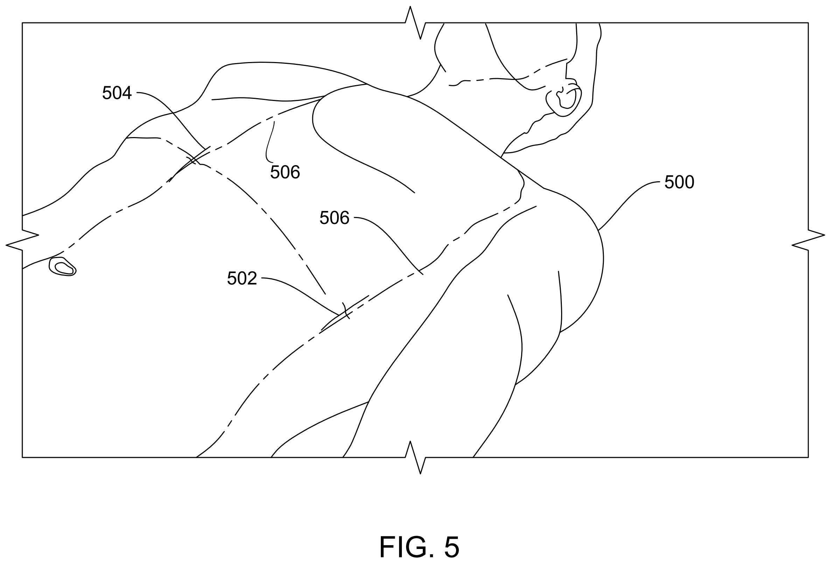

In a non-limiting example, the process begins during the initial imaging session, such as a CT scan, where reference marks are applied to the patient or their immobilization device. For instance, as depicted in **FIG. 5****,** markers **502** and **504** may be placed on the patient **500,** e.g., using tape or other markers. These markers may be subsequently used in the treatment room to correlate with the lasers emitted on the patient **500** (laser beam **506),** ensuring that any shifts or adjustments made during imaging are quantitatively translated into the treatment setup.

During the alignment process, the operator may use laser guidance systems integrated into the treatment room to align the patient's position with the reference marks previously established during the imaging session (markers **502** and **504).** The lasers may present a visual aid that ensures the patient's position on the treatment couch is correctly aligned with the radiotherapy machine's isocenter, thereby maintaining the precision necessary for effective treatment delivery.

Once the laser output (or any other indicator emitted onto the patient, such as any light source) is aligned with the markers, the operator confirms that the emitted laser beam 506 coincides with the reference marks using a button on the pendant or an interactive element on the GUI. This input signals the one or more processors to record that the patient is aligned and ready for subsequent workflow stages and/or tasks. By incorporating this alignment and confirmation process, the disclosed methods and systems ensure consistency, accuracy, and efficiency in patient setup, thereby reducing the risk of errors and enhancing overall treatment quality, and/or improving treatment setup accuracy. This aligning paradigm provides a reliable and repeatable method for maintaining alignment consistency, which is crucial for successful radiotherapy outcomes.

The positions of the reference markers relative to the lasers may provide a measurable framework for making adjustments to the patient's positioning. Operators may use this information to fine-tune the patient's placement on the treatment couch, ensuring alignment with the machine's isocenter.

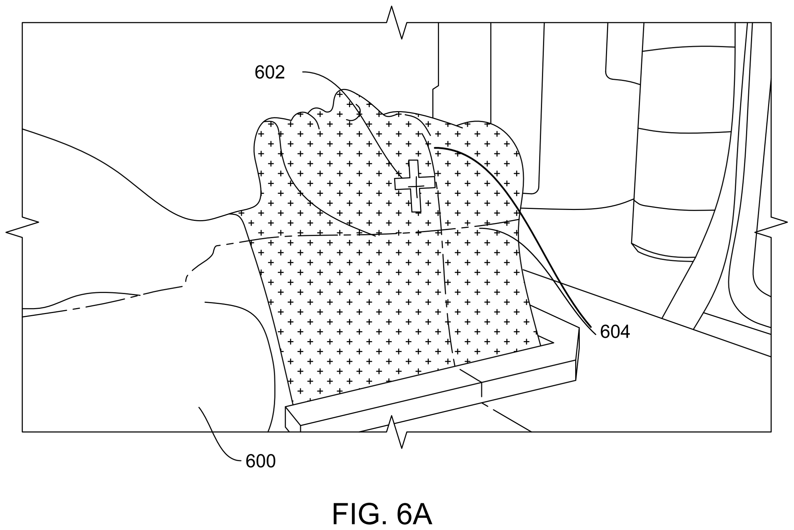

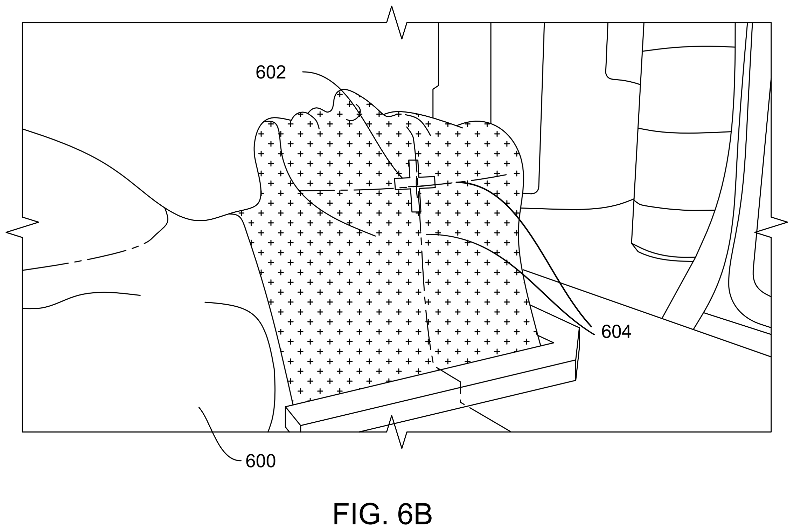

In some embodiments, the markers may be applied to an accessory, such as an immobilization device/mask. This process may be particularly critical for treatment areas such as the head and neck, where immobilization masks are frequently used. For example, as depicted in **FIG. 6A****,** an indicator **602** may be tape or ink used to create visible markers on a mask worn by the patient **600.** The indicator **602** may be designed to align with the laser beam **604** emitted onto the patient **600** in the imaging room. Once the indicator **602** and the laser beam **604** are aligned, as shown in **FIG. 6B****,** the operator may confirm the alignment and the one or more processors may move to the subsequent stage or step of the method **300.**

Referring back to **FIG. 3****,** at step **330,** the one or more processors may present, on the display screen, a visual indicator demonstrating that the radiotherapy machine is aligned for the radiotherapy treatment of the patient. The visual indicator may provide a visual confirmation to the operator that the radiotherapy machine, including components such as the gantry, couch, and/or beam delivery system, is correctly positioned according to the parameters defined in the treatment plan (e.g., greenlighting).

The visual indicator may convey that the radiotherapy machine is in a ready state for treatment because the machine attributes match the desired/preset parameters. As a result, the operator is no longer required to scan multiple attributes to ensure that the machine is ready for treatment. Instead, a single visual indicator conveys that all relevant parameters match the preset/desired attributes for treatment and all the necessary steps have been taken. This greenlighting visual indicator ensures that all necessary machine parameters and/or patient alignment conditions have been properly set before proceeding with treatment.

The visual indicator may take various forms, such as a dedicated icon that indicates proper alignment, a checkmark, a flashing signal, or an animation, to signify successful alignment. For example, a green checkmark icon may appear on the display screen once the alignment is confirmed, signaling that the machine is ready for treatment delivery. In another example, a portion of the GUI may change color (e.g., green is shown on top and bottom or sides of the center portion). Moreover, in some embodiments, a color-coded icon could indicate alignment status, with green signifying successful alignment and red signaling an error or misalignment that requires operator intervention.

In some embodiments, an animated checkmark may appear on the GUI, completing its animation (e.g., status bar) when alignment is verified, providing a visually engaging and clear confirmation for the operator. For instance, a progress bar representing the alignment process could fill completely and/or turn green when the alignment is successfully achieved, offering a clear visual cue.

In some embodiments, the display screen could show a textual confirmation message, such as "Alignment Confirmed" or "Ready for Treatment," accompanied by specific details like the gantry angle or couch position. In some embodiments, the border of the display screen or a designated section (or icon) could flash momentarily and then turn solid to draw the operator's attention to the confirmation of alignment. In some embodiments, a pulsing indicator, such as a circular or rectangular icon, could pulse slowly in green to signal readiness and stability after the alignment is complete. In some embodiments, the system could display a symbolic alignment graphic, such as an icon resembling a radiotherapy machine or gantry, which rotates or animates into place to visually represent the alignment process. In some embodiments, a small sound cue, such as a chime or beep, could accompany the visual indicator, such as a color change or checkmark, to reinforce the confirmation of alignment for the operator.

By revising the GUI to confirm that the patient and/or machine have been properly aligned, the one or more processors can ensure that the operator has a clear and unambiguous confirmation of the machine's readiness, reducing the likelihood of errors and streamlining the treatment workflow.

In some embodiments, the one or more processors may periodically monitor the machine attributes to ensure that they remain at the preset values required for radiotherapy treatment. If any of the monitored machine parameters, such as gantry angle, collimator rotation, couch position, deviate from the preset values, the processors may automatically disable the visual indicator (e.g., the greenlight), to alert the operator that the system is no longer in the ready state. This continuous monitoring ensures that any unintentional adjustments, mechanical drifts, or operator-induced changes are promptly detected, preventing treatment from proceeding under incorrect conditions. By dynamically disabling the visual indicator when deviations occur, the system enhances safety, reinforces treatment accuracy, and ensures that the operator is aware of the need for corrective realignment before proceeding with patient treatment.

The visual indicator can signify that all (or a portion of) the attributes correspond to a defined attribute, such as the couch position matching the required position for the treatment to start. This single visual indicator can confirm that multiple other attributes are satisfied, eliminating the need for the operator to check each attribute individually. For instance, a single visual indicator can mean that the couch, gantry, and collimator are in proper position and condition for the treatment to start. Therefore, the operator is no longer required to check the attributes of the couch, gantry, or collimator individually. This is particularly beneficial as it simplifies the process, reducing the need for the operator to navigate through multiple places to verify different attributes. By providing a clear and comprehensive visual cue, the system enhances efficiency and reduces the cognitive load on the operator, ensuring that the machine is ready for treatment with minimal effort.

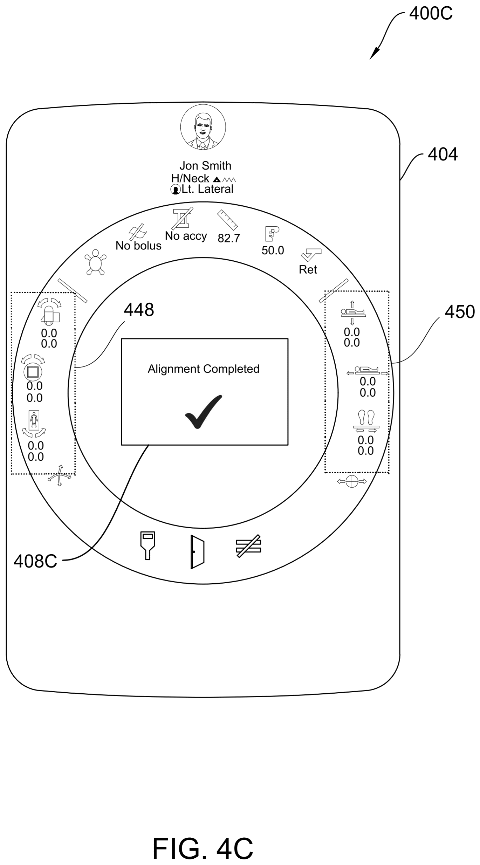

Referring to **FIG. 4C****,** the one or more processors may revise the center portion **408C** to display a message indicating that the alignment has been completed. The message may also include a checkmark (or any other dedicated visual marking in any defined color) indicating that the alignment stage has been successfully completed.

Referring back to **FIG. 3****,** at step **340,** the one mor more processors may configure the at least one attribute of the radiotherapy machine as a default attribute. This concept is also referred to as "setting," "resetting," or "zeroing" the radiotherapy machine and ensures that the machine is initialized with predefined settings that align with standard operational requirements or specific treatment protocols. The default attribute may encompass various operational parameters of the radiotherapy machine, such as the gantry angle, couch position, beam intensity, dose delivery settings, or imaging configurations. For example, the one or more processors may configure the gantry to a default angle of 0 degrees or set the couch to a default height that corresponds to the standard treatment isocenter or 0 inches. These defaults may be based on standard practices, treatment planning data, or operator-defined preferences stored in the system. Any further adjustments will be measured from this new default position based on the at least one attribute.

The ability to configure default attributes ensures that the radiotherapy machine begins the treatment session in a consistent and predictable state. Additionally, operators may have the flexibility to customize these default settings, tailoring them to specific patient needs or clinical workflows. This functionality improves operational efficiency, enhances safety, and minimizes the cognitive load on operators during the preparation and delivery of radiotherapy treatments.

In some embodiments, the GUI may also be dynamically revised to indicate that the machine configurations have been set or reset to the default configuration. Referring again to **FIG. 4C****,** the display screen **404** may be updated such that the values indicated for the alignment icons (e.g., icons **412, 414, 416, 428, 430,** and **432)** have been set to zero (regions **448** and **450).** In some embodiments, these icons (or a portion of the icon, such as the arrow) may also change in color (e.g., green) to indicate that no additional changes are needed and the alignment has been set to default.

In some embodiments, the operator may be required to make additional alignments to the patient and/or modifications to the machine after the machine has been "zeroed." As a result, the additional changes may be reflected on the GUI in terms of value after the machine has been "zeroed." Zeroing establishes a baseline reference point for all positional measurements, allowing subsequent adjustments to be made relative to this origin. However, during the course of treatment setup or alignment verification, the operator may identify the need for further refinements, such as repositioning the patient on the treatment couch, altering the gantry angle, or making small couch shifts. These adjustments ensure that the alignment remains precise, accommodating any unforeseen discrepancies or changes that arise after the initial zeroing process.

To account for these additional changes, the one or more processors may dynamically update the GUI to reflect the new values. For instance, if the couch is moved slightly after being zeroed, the GUI may display the new positional offset relative to the zeroed baseline.

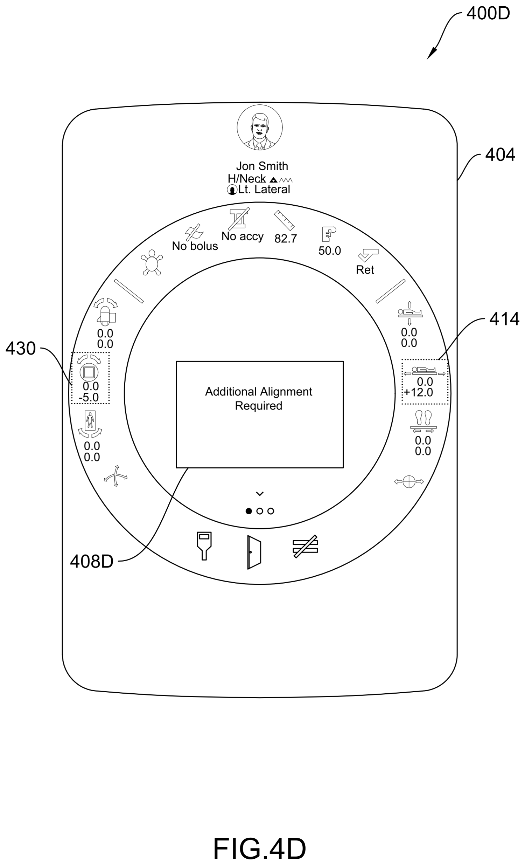

Referring now to **FIG. 4D****,** embodiment **400D** depicts an example where the machine has been zeroed before additional changes are made. As a result, the center portion **408D** is dynamically revised to indicate that additional alignments are required. Moreover, values for icons **414** and **430** are revised to reflect couch position and gantry angles relative to the zeroed or default position. As depicted, the alignment values are with respect to the zeroed position (e.g., default configuration) and not the original position with which the values depicted in **FIG. 4A** were calculated. This approach allows operators to visualize and track all subsequent adjustments clearly, maintaining an accurate and consistent alignment framework. By reflecting these modifications in real time, the system ensures transparency and reduces the likelihood of errors, ultimately enhancing precision and confidence during critical stages of radiotherapy treatment setup.

In some embodiments, one or more settings and attributes, as configured using the methods discussed herein, can be saved to ensure consistency and efficiency across multiple treatment sessions. By storing the configured attributes, such as gantry angle, couch position, beam intensity, or calibration parameters, the radiotherapy machine can be initialized with the same configuration at the start of each subsequent session. This capability eliminates the need for repetitive setup processes, reducing operator workload and minimizing the potential for errors during reconfiguration. Additionally, saving these settings ensures that treatment parameters remain aligned with the patient's treatment plan, enabling seamless continuity across sessions. This functionality is particularly beneficial in cases where precise and repeatable configurations are critical for achieving optimal treatment outcomes, such as in multi-fraction radiotherapy regimens. By allowing operators to retrieve and apply saved configurations, the system enhances workflow efficiency, supports high-precision treatment setup and supports high-precision treatment delivery.

Though zeroing is described herein in the context of a default position of the machine that matches the aligned position (e.g., where all the parameters match preset machine attributes needed to start the treatment), the operator can "zero" the machine at any desired attribute or position. For instance, the default position may be defined by the operator at a position that does not match the preset conditions/attributes needed to start the treatment. In those embodiments, the machine the visual indicator (e.g., green light) will not be displayed; however, the parameters may be set to zero.

In some embodiments, the zeroing may be performed at any time, such as any time after at least one attribute of the radiotherapy machine and/or patient has been adjusted. For instance, at the start of the setup, the machine is assumed to be at a zero position. After at least one parameter has been changed (e.g., a couch has been moved), the operator can perform the zeroing. Referring back to **FIG. 3****,** the operator may be presented with a set of instructions associated with adjusting at least one attribute of the radiotherapy machine before the radiotherapy treatment starts, as discussed with respect to step **310.** That is, various icons discussed herein may indicate the adjustments needed to be made. Similar to the step 320, the operator may adjust one or more parameters and the one or more processors may receive an input (e.g., via the pendant) that a change has been made.

The one or more processors may then receive an instruction from the operator to perform "zeroing." Specifically, the one or more processors may receive an instruction to set the machine parameter to a default attribute. As discussed herein, the instruction can be issued by the operator at any point during the radiotherapy treatment/setup process. The operator can use a variety of input methods to instruct the one or more processors to perform zeroing.

In some embodiments, the operator can use a pendant connected to the radiotherapy machine to issue instructions. The pendant may include buttons or a touchpad that allows the operator to navigate through the machine's settings and select the option to reset the parameters to the default attribute. In some embodiments, the operator can interact with a dedicated button on the pendant and/or on the user interface to confirm the alignment and set the machine parameters to zero. In another example, the operator may be able to use voice commands to issue instructions to the machine. The radiotherapy machine can be equipped with voice recognition technology that allows the operator to verbally instruct the machine to reset the parameters to the default attribute.

Upon receiving this instruction, the one or more processors may configure at least one attribute of the radiotherapy machine as a default attribute. For instance, the one or more processors may reset the specified machine parameter, such as the gantry angle, couch position, or collimator rotation, or sometimes all the parameters to a predefined default value (e.g., 0 value). This default attribute may serve as a standardized reference point, ensuring consistency and accuracy in the machine's configuration.

### Example clauses

Further aspects of these teachings are provided by the subject matter of the following clauses.

Clause 1. A computer-readable medium comprising a set of instructions, that when executed, cause at least one processor to: present, on a display screen located on a gantry of a radiotherapy machine configured to implement radiotherapy treatment of a patient, a set of icons corresponding to a set of instructions associated with adjusting at least one attribute of the radiotherapy machine before the radiotherapy treatment starts; receive an input that the set of instructions have been satisfied; present, on the display screen, a visual indicator demonstrating that the radiotherapy machine is aligned for the radiotherapy treatment of the patient.

Clause 2. The computer-readable medium of clause 1, wherein the input is received via an interaction with the display screen.

Clause 3. The computer-readable medium of clauses 1-2, wherein the input is received via a pendant of the radiotherapy machine.

Clause 4. The computer-readable medium of clauses 1-3, wherein the pendant includes a button for the input.

Clause 5. The computer-readable medium of clauses 1-4, wherein the visual indicator is an additional icon displayed on the display screen.

Clause 6. The computer-readable medium of clauses 1-5, wherein the visual indicator corresponds to a change of a visual attribute for at least one icon within the set of icons.

Clause 7. The computer-readable medium of clauses 1-6, wherein the input is received after aligning a marking on a patient with an indicator emitted on the patient.

Clause 8. The computer-readable medium of clauses 1-7, wherein the set of instructions further cause the at least one processor to: identify an additional adjustment to the at least one attribute; and present the additional adjustment in terms of a default configuration and not an original radiotherapy configuration.

Clause 9. A computer system comprising: a radiotherapy machine; and at least one processor in communication with the radiotherapy machine, the at least one processor configured to: present, on a display screen located on a gantry of the radiotherapy machine configured to implement radiotherapy treatment of a patient, a set of icons corresponding to a set of instructions associated with adjusting at least one attribute of the radiotherapy machine before the radiotherapy treatment starts; receive an input that the set of instructions have been satisfied; present, on the display screen, a visual indicator demonstrating that the radiotherapy machine is aligned for the radiotherapy treatment of the patient.

Clause 10. The computer system of clause 9, wherein the input is received via an interaction with the display screen.

Clause 11. The computer system of clauses 9-10, wherein the input is received via a pendant of the radiotherapy machine.

Clause 12. The computer system of clauses 9-11, wherein the pendant includes a button for the input.

Clause 13. The computer system of clauses 9-12, wherein the visual indicator is an additional icon displayed on the display screen.

Clause 14. The computer system of clauses 9-13, wherein the visual indicator corresponds to a change of a visual attribute for at least one icon within the set of icons.

Clause 15. The computer system of clauses 9-14, wherein the input is received after aligning a marking on a patient with an indicator emitted on the patient.

Clause 16. The computer system of clauses 9-15, wherein the at least one processor is further configured to: identify an additional adjustment to the at least one attribute; and present the additional adjustment in terms of a default configuration and not an original radiotherapy configuration.

Clause 17. A computer-readable medium comprising a set of instructions, that when executed, cause at least one processor to: present, on a display screen located on a gantry of a radiotherapy machine configured to implement radiotherapy treatment of a patient, a set of icons corresponding to a set of instructions associated with adjusting at least one attribute of the radiotherapy machine before the radiotherapy treatment starts; receive an instruction to set a machine parameter to a default attribute; and configure the at least one attribute of the radiotherapy machine as the default attribute.

Clause 18. The computer-readable medium of clause 17, wherein the instruction is received via an interaction with the display screen.

Clause 19. The computer-readable medium of clauses 17-18, wherein the instruction is received via a pendant of the radiotherapy machine.

Clause 20. The computer-readable medium of clauses 17-19, wherein the pendant includes a button for the instruction.

Clause 21. A method to execute the steps carried out by the at least one processor of the computer-readable medium of any of clauses 1-8.

Clause 22. A method to execute the steps of the computer system of any of clauses 9-16.

Clause 23. A method to execute the steps carried out by the at least one processor of the computer-readable medium of any of clauses 17-20.

Clause 24. A computer system comprising:
a radiotherapy machine; and
at least one processor in communication with the radiotherapy machine, the at least one processor configured to:
   present, on a display screen located on a gantry of a radiotherapy machine configured to implement radiotherapy treatment of a patient, a set of icons corresponding to a set of instructions associated with adjusting at least one attribute of the radiotherapy machine before the radiotherapy treatment starts;
   receive an instruction to set a machine parameter to a default attribute; and
   configure the at least one attribute of the radiotherapy machine as the default attribute.

Clause 25. The computer system of clause 24, wherein the instruction is received via an interaction with the display screen.

Clause 26. The computer system of clause 24 or clause 25, wherein the instruction is received via a pendant of the radiotherapy machine.

Clause 27. The computer system of any of clause 24 to clause 26, wherein the pendant includes a button for the instruction.

The presentation of information on the display screen, the user interaction with the display screen and/or the GUI, may have the technical effect of assisting the user in performing a radiotherapy setup workflow by means of a continued and/or guided process of human-machine interaction. The systems discussed herein may be configured to assist the user in performing a radiotherapy workflow and/or a radiotherapy setup workflow, by means of a continued and/or guided process of human-machine interaction.

In this disclosure, the term "radiotherapy treatment" may refer to radiotherapy treatment and/or radiotherapy treatment setup. The method steps may refer to treatment setup, while the systems may be configured to perform treatment and/or treatment setup.

The foregoing method descriptions and the process flow diagrams are provided merely as illustrative examples and are not intended to require or imply that the steps of the various embodiments must be performed in the order presented. The steps in the foregoing embodiments may be performed in any order. Words such as "then," "next," etc. are not intended to limit the order of the steps; these words are simply used to guide the reader through the description of the methods. Although process flow diagrams may describe the operations as a sequential process, many of the operations can be performed in parallel or concurrently. In addition, the order of the operations may be re-arranged. A process may correspond to a method, a function, a procedure, a subroutine, a subprogram, and the like. When a process corresponds to a function, the process termination may correspond to a return of the function to a calling function or a main function.

The various illustrative logical blocks, modules, circuits, and algorithm steps described in connection with the embodiments disclosed herein may be implemented as electronic hardware, computer software, or combinations of both. To clearly illustrate this interchangeability of hardware and software, various illustrative components, blocks, modules, circuits, and steps have been described above generally in terms of their functionality. Whether such functionality is implemented as hardware or software depends upon the particular application and design constraints imposed on the overall system. Skilled artisans may implement the described functionality in varying ways for each particular application, but such implementation decisions should not be interpreted as causing a departure from the scope of the present invention.

Embodiments implemented in computer software may be implemented in software, firmware, middleware, microcode, hardware description languages, or any combination thereof. A code segment or machine-executable instructions may represent a procedure, a function, a subprogram, a program, a routine, a subroutine, a module, a software package, a class, or any combination of instructions, data structures, or program statements. A code segment may be coupled to another code segment or a hardware circuit by passing and/or receiving information, data, arguments, parameters, or memory contents. Information, arguments, parameters, data, etc. may be passed, forwarded, or transmitted via any suitable means including memory sharing, message passing, token passing, network transmission, etc.

The actual software code or specialized control hardware used to implement these systems and methods is not limiting of the invention. Thus, the operation and behavior of the systems and methods were described without reference to the specific software code being understood that software and control hardware can be designed to implement the systems and methods based on the description herein.

When implemented in software, the functions may be stored therein as one or more instructions or code on a computer-readable, non-transitory medium or processor-readable storage medium. The steps of a method or algorithm disclosed herein may be embodied in a processor-executable software module, which may reside on a computer-readable or processor-readable storage medium. A non-transitory computer-readable or processor-readable media includes both computer storage media and tangible storage media that facilitate transfer of a computer program from one place to another. A non-transitory processor-readable storage media may be any available media that may be accessed by a computer. By way of example, and not limitation, such non-transitory processor-readable media may comprise RAM, ROM, EEPROM, CD-ROM or other optical disk storage, magnetic disk storage or other magnetic storage devices, or any other tangible storage medium that may be used to store desired program code in the form of instructions or data structures and that may be accessed by a computer or processor. Disk and disc, as used herein, include compact disc (CD), laser disc, optical disc, digital versatile disc (DVD), floppy disk, and Blu-ray disc where disks usually reproduce data magnetically, while discs reproduce data optically with lasers. Combinations of the above should also be included within the scope of computer-readable media. Additionally, the operations of a method or algorithm may reside as one or any combination or set of codes and/or instructions on a non-transitory processor-readable medium and/or computer-readable medium, which may be incorporated into a computer program product.

The preceding description of the disclosed embodiments is provided to enable any person skilled in the art to make or use the present invention. Various modifications to these embodiments will be readily apparent to those skilled in the art, and the generic principles defined herein may be applied to other embodiments without departing from the scope of the invention. Thus, the present invention is not intended to be limited to the embodiments shown herein but is to be accorded the widest scope consistent with the following claims and the principles and novel features disclosed herein.

While various aspects and embodiments have been disclosed, other aspects and embodiments are contemplated. The various aspects and embodiments disclosed are for purposes of illustration and are not intended to be limiting, with the true scope being indicated by the following claims.

## Claims

1. A computer-readable medium comprising a set of instructions, that when executed, cause at least one processor to:
present, on a display screen coupled to a radiotherapy machine configured to implement radiotherapy treatment of a patient, a set of icons corresponding to a set of instructions associated with adjusting at least one attribute of the radiotherapy machine before the radiotherapy treatment starts;
receive an input that the set of instructions have been satisfied;
present, on the display screen, a visual indicator demonstrating that the radiotherapy machine is aligned for the radiotherapy treatment of the patient.

2. The computer-readable medium of claim 1, wherein the input is received via an interaction with the display screen.

3. The computer-readable medium of claim 1 or claim 2, wherein the input is received via a pendant of the radiotherapy machine;
and optionally:
wherein the pendant includes a button for the input.

4. The computer-readable medium of any preceding claim, wherein the visual indicator is an additional icon displayed on the display screen;
and/or:
wherein the visual indicator corresponds to a change of a visual attribute for at least one icon within the set of icons.

5. The computer-readable medium of any preceding claim, wherein the input is received after aligning a marking on a patient with an indicator emitted on the patient.

6. The computer-readable medium of any preceding claim, wherein the set of instructions further cause the at least one processor to:
identify an additional adjustment to the at least one attribute; and
present the additional adjustment in terms of a default configuration and not an original radiotherapy configuration.

7. A computer system comprising:
a radiotherapy machine; and
at least one processor in communication with the radiotherapy machine, the at least one processor configured to:
present, on a display screen coupled to the radiotherapy machine configured to implement radiotherapy treatment of a patient, a set of icons corresponding to a set of instructions associated with adjusting at least one attribute of the radiotherapy machine before the radiotherapy treatment starts;
receive an input that the set of instructions have been satisfied;
present, on the display screen, a visual indicator demonstrating that the radiotherapy machine is aligned for the radiotherapy treatment of the patient.

8. The computer system of claim 7, wherein the input is received via an interaction with the display screen.

9. The computer system of claim 7 or claim 8, wherein the input is received via a pendant of the radiotherapy machine;
and optionally:
wherein the pendant includes a button for the input.

10. The computer system of any of claim 7 to claim 9, wherein the visual indicator is an additional icon displayed on the display screen;
and/or:
wherein the visual indicator corresponds to a change of a visual attribute for at least one icon within the set of icons.

11. The computer system of any of claim 7 to claim 11, wherein the input is received after aligning a marking on a patient with an indicator emitted on the patient;
and/or:
wherein the at least one processor is further configured to:
identify an additional adjustment to the at least one attribute; and
present the additional adjustment in terms of a default configuration and not an original radiotherapy configuration.

12. A computer-readable medium comprising a set of instructions, that when executed, cause at least one processor to:
present, on a display screen coupled to a radiotherapy machine configured to implement radiotherapy treatment of a patient, a set of icons corresponding to a set of instructions associated with adjusting at least one attribute of the radiotherapy machine before the radiotherapy treatment starts;
receive an instruction to set a machine parameter to a default attribute; and
configure the at least one attribute of the radiotherapy machine as the default attribute.

13. The computer-readable medium of claim 12, wherein the instruction is received via an interaction with the display screen.

14. The computer-readable medium of claim 12 or claim 13, wherein the instruction is received via a pendant of the radiotherapy machine.

15. The computer-readable medium of claim 14, wherein the pendant includes a button for the instruction.
